Europäisches Patentamt

(19) European Patent Office

Office européen des brevets

(11) EP 0 836 381 B1

## (12) EUROPEAN PATENT SPECIFICATION

(45) Date of publication and mention
of the grant of the patent:
**29.09.1999 Bulletin 1999/39**

(21) Application number: **96920517.8**

(22) Date of filing: **24.05.1996**

(51) Int. Cl.⁶: **A01N 27/00**, C12N 15/82

(86) International application number:
**PCT/US96/07768**

(87) International publication number:
**WO 96/37102** (28.11.1996 Gazette 1996/52)

(54) **CORN ROOTWORM CONTROL VIA FORMATION OF LIMONENE IN TRANSGENIC PLANTS**

BEKÄMPFUNG VON DIABROTICA UNDECIMPUNCTATA MITTELS LIMONENBILDUNG IN
TRANSGENEN PFLANZEN

LUTTE CONTRE LA CHRYSOMELE MACULEE DU CONCOMBRE PAR LA FORMATION DE
LIMONENE DANS DES PLANTES TRANSGENIQUES

(84) Designated Contracting States:
**AT BE CH DE DK ES FI FR GB GR IE IT LI LU MC
NL PT SE**

(30) Priority: **24.05.1995 US 449061**

(43) Date of publication of application:
**22.04.1998 Bulletin 1998/17**

(73) Proprietor:
**PIONEER HI-BRED INTERNATIONAL, INC.
Des Moines, Iowa 50309 (US)**

(72) Inventor: **MEYER, Terry, E.
Urbandale, IA 50322 (US)**

(74) Representative:
**Goldin, Douglas Michael et al
J.A. KEMP & CO.
14 South Square
Gray's Inn
London WC1R 5LX (GB)**

(56) References cited:
**WO-A-94/22304**

## Description

### Technical Field

This invention relates to providing maize and other plants which produce no or low levels of limonene with resistance to Southern Corn Rootworm and other insect pests by causing expression of limonene in the tissues of the plants in quantities sufficient to repel, kill, or inhibit those pests.

### Background of the Invention

Numerous insects are serious pests of common agricultural crops. One method of controlling insects has been to apply insecticidal organic, semiorganic or organometallic chemicals to crops. This method has numerous, art-recognized environmental and public health problems. A more recent method of control of insect pests has been the use of biological control organisms which are typically natural predators of the troublesome insects. These include other insects such as trachonid wasps, fungi such as milky-spore fungi, and bacteria such as Bacillus thuringiensis cv., commonly referred to as "Bt". However, it is difficult to apply biological control organisms to large areas, and even more difficult to cause those living organisms to remain in the treated area for an extended period. Still more recently, techniques in recombinant DNA have provided the opportunity to insert into plant cells cloned genes which express insecticidal toxins derived from biological control organisms such as Bt. This technology has given rise to additional concerns about eventual insect resistance to well-known, naturally occurring insect toxins, particularly in the face of heavy selection pressure, which may occur in some areas. Thus, a continuing need exists to identify naturally occurring insecticidal toxins which can be formed by plant cells directly by expression of a single structural gene which is not normally present in the plant.

Southern Corn Rootworm (Diabrotica undecimpunctata howardi Barber) is a particularly difficult pest to control or eradicate. It attacks the plant below the soil line, where insecticides are difficult or impossible to apply effectively. In addition, it is resistant to a number of otherwise effective chemical and biological control agents, including Bt toxins and some lectins.

Limonene, 1-methyl-4-(1-methylethenyl)cyclohexene; p-mentha-1,8-diene (Entry No. 5371, Merck Index 11th Ed.), occurs naturally in various ethereal oils, particularly oils of lemon, orange, caraway, dill and bergamot. It is a valuable industrial chemical. Some limonene is prepared by extraction from plants of the mint family, a large quantity is obtained from citrus oils, which are typically 80-90% limonene, and some is obtained from pine oil. It is also synthesized chemically and finds use as a solvent and cleaning agent (in the manufacture of synthetic pine oil), as an expectorant, as a wetting and dispersing agent, as a monomer in the manufacture of various polymeric resins, as a flavorant and a precursor in the synthesis of the flavorant carvone, and as a polymerization inhibitor in storage of the tetrafluoroethylene monomer used in the manufacture of polytetrafluoroethylene (PTFE).

WO-A-9422304 discloses the use of limonene in controlling plant pests of Diabrotica spp. such as Southern Corn Rootworm. One of the disclosed methods for effecting this is the introduction of a gene sequence coding for limonene cyclase into plant cells, via known methods. The sequence is inserted into an appropriate expression cassette. Preferably, a DNA sequence coding for limonene synthase is inserted into a plant or bacterial cell in proper reading frame relative to transcription initiator and promoter sequences active in the plant or bacterium.

### DISCLOSURE OF THE INVENTION

The invention relates to a method for protecting a monocotyledonous plant against infestation by larvae of Diabrotica spp comprising inserting into the genome of the plant at least one nucleotide sequence coding for limonene cyclase and at least one nucleotide sequence coding for GPP synthase, each of said sequences in proper reading frame relative to transcription initiator and promoter sequences active in the plant to cause expression of the enzymes at levels that provide for production of a plant protective effective amount of limonene in the tissues of the plant.

By "transgenic plant" is meant any plant or plant cell that has become transformed by the introduction, stable and heritable incorporation, into the subject plant or plant cell, of either native DNA that is under the control of a promoter other than the promoter that typically drives expression of that DNA in a wild-type plant, and that has been introduced back into its host plant, or foreign DNA, i.e. DNA encoding for a protein not normally found within that plant species.

"Plantlet" refers to a plant sufficiently developed to have a shoot and a root that is asexually reproduced by cell culture.

"Explant" refers to a section or piece of tissue from any part of a plant for culturing.

The term "callus" and its plural "calli", refer to an unorganized group of cells formed in response to cutting, severing, or other injury inflicted on plant tissue. Excised pieces of plant tissue and isolated cells can be induced to form

EP 0 836 381 B1

callus under the appropriate culture conditions. Callus can be maintained in culture for a considerable time by transferring or subculturing parts of the callus to fresh medium at regular intervals. The transfer of callus to liquid medium leads to dispersion of the tissue and the formation of a plant cell suspension culture. Callus can be induced to undergo organized development to form shoots and roots.

"Embryoid" refers to a structure similar in appearance to a plant Zygotic embryo.

By the term "taxon" herein is meant a unit of botanical classification of genes or lower. It thus includes genus, species, cultivar, variety, variant, and other minor taxonomic groups that lack a consistent nomenclature.

"Somatic hybrid" and "somatic hybridization" refers generally to stable combination of cellular material, be it protoplast/protoplast or protoplast/cytoplast combinations, and includes cybrids and cybridization.

A "replicon" is any genetic element (e.g., plasmid, chromosome, virus) that functions as an autonomous unit of DNA replication in vivo; i.e., capable of replication under its own control.

As used herein, the term "nucleotide sequence" means a DNA or RNA sequence, and can include a cDNA, or genomic DNA, or synthetic DNA sequence, a structural gene or a fragment thereof, or an mRNA sequence, that encodes an active or functional polypeptide.

A "vector" is a replicon, such as a plasmid, phage, or cosmid, to which another DNA segment may be attached so as to bring about the replication of the attached segment.

A DNA "coding sequence" is a DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, procaryotic sequences, cDNA from eucaryotic mRNA, genomic DNA sequences from eucaryotic (e.g., mammalian) DNA, and even synthetic DNA sequences. A polyadenylation signal and transcription termination sequence will usually be located 3' to the coding sequence.

A "promoter sequence" or a "promoter" is a DNA regulatory region capable of binding RNA polymerase in a cell and initiating transcription of a downstream (3' direction) coding sequence. For purposes of defining the present invention, the promoter sequence is bound at its 3' terminus by the translation start codon (ATG) of a coding sequence and extends upstream (5' direction) to include the minimum number of bases or elements necessary to initiate transcription. Within the promoter sequence will be found a transcription initiation site, as well as protein binding domains responsible for the binding of RNA polymerase. Eucaryotic promoters will often, but not always, contain "TATA" boxes and "CAT" boxes.

DNA "control sequences" refers collectively to promoter sequences, ribosome binding sites, polyadenylation signals, transcription termination sequences, upstream regulatory domains, enhancers, and the like, which collectively provide for the transcription and translation of a coding sequence in a host cell.

A coding sequence is "operably linked to" or "under the control of" control sequences in a cell when RNA polymerase will bind the promoter sequence and transcribe the mRNA, which is then translated into the polypeptide encoded by the coding sequence.

A "host cell" is a cell which has been transformed, or is capable of undergoing transformation, by an exogenous DNA sequence.

A cell has been "transformed" by endogenous or exogenous DNA when such DNA has been introduced inside the cell membrane. The DNA may or may not be integrated into (covalently linked to) chromosomal DNA making up the genome of the transformed cell. In procaryotes and yeasts, for example, the DNA may be maintained on an episomal element, such as a plasmid. With respect to eucaryotic cells, a stably transformed cell is one in which the DNA has become integrated into the chromosome so that it is inherited by daughter cells through chromosome replication. This stability is demonstrated by the ability of the eucaryotic cell to establish cell lines or clones comprised of a population of daughter cells containing the DNA.

A "clone" is a population of cells derived from a single cell or common ancestor by mitosis. A "cell line" is a clone of a primary cell that is capable of stable growth in vitro for many generations.

Two DNA, RNA or polypeptide sequences are "substantially homologous" when at least about 85% (preferably at least about 90%, and most preferably at least about 95%) of the nucleotides or amino acids match over a defined length of the molecule. DNA sequences that are substantially homologous can be identified in a Southern hybridization experiment under, for example, stringent conditions, as defined for that particular system. Defining appropriate hybridization conditions is within the skill of the art. See, e.g., Maniatis et al., Molecular Cloning: A Laboratory Manual, Cold Spring Harbor Laboratory, New York, 1982; Brown, T. A., Gene Cloning: An Introduction (2nd Ed.) Chapman & Hall, London (1990).

A "heterologous" region of a DNA construct is an identifiable segment of DNA within or attached to another DNA molecule that is not found in association with the other molecule in nature. Thus, when the heterologous region encodes a bacterial gene, the gene will usually be flanked by DNA that does not flank the bacterial gene in the genome of the source bacterium. Another example of a heterologous coding sequence is a construct where the coding sequence itself is not found in nature (e.g., synthetic sequences having codons different from the native gene). "Heter-

3

ologous" DNA also refers to DNA not found within the host cell in nature. Allelic variation or naturally occurring mutational events do not give rise to a heterologous region of DNA, as these terms are used herein. "Native", "autologous" or "endogenous" DNA, as used herein, refer to DNA that is typically present in the host in nature.

The term "polypeptide" as used herein is used in its broadest sense, i.e., any polymer of amino acids (dipeptide or greater) linked through peptide bonds. Thus, the term "polypeptide" includes proteins, oligopeptides, protein fragments, analogues, muteins, fusion proteins and the like. The term also encompasses amino acid polymers as described above that include additional non-amino acid moieties. Thus, the term "polypeptide" includes glycoproteins, lipoproteins, phosphoproteins, metalloproteins, nucleoproteins, as well as other conjugated proteins. The term "polypeptide" contemplates polypeptides as defined above that are recombinantly produced, isolated from an appropriate source, or synthesized.

In view of the ability to transform crop plants to express various heterologous compounds, it would be desirable to transform maize plants to express limonene, so that by consuming the tissues of the plant the larvae would also consume toxic or inhibitory amounts of limonene. However, while most gene products are peptides, limonene is not a peptide or a peptide derivative and is not expressed from genes in the form of a peptide or peptide derivative, but is produced enzymatically as a secondary metabolite within the cells of some plants. We have now also found that the biosynthetic apparatus necessary for the production of limonene may not be present in many plant cells which do not produce limonene, or may not produce detectable, insecticidally effective amounts of limonene, and this appears to include maize cells. Such plant cells must be engineered with at least one enzyme which can be produced through the expression of exogenous (heterologous) genes. One such enzyme is limonene synthase, also known as limonene cyclase, which can directly synthesize limonene from geranyl pyprophosphate (GPP), which is found widely in both procaryotic and eucaryotic cells, although, as will be discussed, is in some cases not produced in quantities sufficient to make insecticidally effective amounts of limonene. Since genes which code for limonene synthase can be synthesized, either directly using a DNA sequence obtained by working backwards from the known amino acid sequence of limonene synthase and preferably using plant-preferred codons, or by cloning from natural sources of limonene, the resulting sequence can be inserted into an appropriate expression cassette, and introduced into cells of a susceptible plant species or a suitable endophytic bacterium, so that an especially preferred embodiment of this method involves inserting into the genome of the plant or bacterium a DNA sequence coding for limonene synthase, in proper reading frame relative to transcription initiator and promoter sequences active in the plant or bacterium. Transcription and translation of the DNA sequence under control of the regulatory sequences, can, in some cases, cause expression of the enzyme at levels which provide an insecticidal amount of limonene in the tissues of the plant which are normally infested by the larvae.

As an illustration, it can be noted that Colby et al., at the Keystone Symposium on Crop Improvement via Biotechnology: An International Perspective, Keystone, Colorado, USA, April 10-16, 1992, as reported in J. Cell Biochem. Suppl. 16, F, 230 (1992) have isolated and characterized cDNA encoding limonene cyclase from spearmint. To isolate and study the gene(s) (sic) encoding limonene synthase and to produce enough of the enzyme for structural studies, they used standard methods to extract RNA from young leaves of Mentha spicata and constructed a cDNA library in lamoda ZAP XR (Stratagene) from poly (A)+ RNA. They designed three degenerate oligonucleotides based on internal amino acid sequences obtained from Edman degradation of purified limonene synthase and screened 250,000 clones to identify six positive clones that hybridized to all three oligonucleotides. The resulting clones could be used in the methods of this invention which involve plant transformation. However, Colby et al. indicate no appreciation of the value of the enzyme in conferring rootworm resistance to plants.

Additionally, it has been discovered that in certain plants, including maize, at least one additional gene, that encoding GPP synthase, is required for generation of rootworm-resistant plants. Due to the fact that natural levels of GPP are low in such plants, there may be inadequate amounts of GPP for limonene production in maize or other plants that are transformed solely with the limonene synthase gene. This is in contrast to GPP levels in other plant species, such as spearmint, which are much higher. In such species, some GPP is used to generate limonene, some to generate other metabolites. Because GPP is derived from a pathway that is common among plant species, the introduction of both the GPP synthase gene and the limonene synthase gene into plant species such as maize can produce transgenic plants capable of expressing GPP, and then the metabolic product limonene, at levels sufficient to confer resistance to insect pests, particularly corn rootworm.

The plant which can be benefited by this invention is preferably a plant susceptible to infestation and damage by the larvae of Diabrotica undecimpunctata howardi or whose harvested material is subject to attack by larvae of that insect. A prime example is corn (Zea mays). Transformation of maize cells, and the regeneration of transformed cells to produce whole, fertile, transformant (R0) offspring has also been reported by several centers and is now routine. However, this is not to be construed as limiting, inasmuch as this species has in the past been among the most difficult commercial crops to reliably transform and regenerate, and these insects (under other common names) also infest certain other crops. Thus the methods of this invention are readily applicable via conventional techniques to numerous plant species, if they are found to be susceptible to Diabrotica undecimpunctata howardi, including, without limitation, species from the genera *Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Triticum,* and *Datura.*

Preferred plants that are to be transformed according to the methods of this invention are cereal crops, including maize, rye, barley, wheat, sorghum, oats, millet, rice, triticale, sunflower, alfalfa, rapeseed and soybean, fiber crops, such as cotton, fruit crops, such as melons, and vegetable crops, including onion, pepper, tomato, cucumber, squash, carrot, crucifer (cabbage, broccoli, cauliflower), eggplant, spinach, potato and lettuce.

The DNA sequence or sequences which when expressed impart insecticidal activity are either a structural gene which codes for limonene synthase, or a combination of that structural gene and a structural gene that encodes GPP synthase, depending upon the level of GPP produced in the wild type plant. It has been found that limonene has sufficient insecticidal (larvicidal) activity to be operative when formed as the end product of a plant cell expression system. That is, while certain other compounds have some larvicidal activity at high concentrations in pure form, plant cell expression at such high concentrations is either not possible in a living plant cell system, or is not feasible if the commercially useful characteristics of the plant are to be preserved in terms of production of oils, starches, fibers, or other materials. Limonene, on the other hand, is not directly expressed as the gene product, and the peptide or peptides which is or are expressed in the methods of this invention is an enzyme which can catalyze the synthesis of large amounts of limonene for accumulation in the tissues of the transformed plant (limonene synthase), and in instances in which it is required, an enzyme which can catalyze the synthesis of large amounts of the substrate for limonene synthase.

A tissue-specific promoter (or promoters) can be used in any instance where it may be desirable to localize production of limonene to an infested tissue or to a tissue which is efficient in production of the enzyme. Since Southern corn rootworm attack roots, an especially preferred tissue-specific promoter is a root-specific promoter.

In carrying out this invention, it will be appreciated that numerous plant expression cassettes and vectors are well known in the art. By the term "expression cassette" is meant a complete set of control sequences including initiation, promoter and termination sequences which function in a plant cell when they flank a structural gene in the proper reading frame. Expression cassettes frequently and preferably contain an assortment of restriction sites suitable for cleavage and insertion of any desired structural gene. It is important that the cloned gene have a start codon in the correct reading frame for the structural sequence. In addition, the plant expression cassette preferably includes a strong promoter sequence at one end to cause the gene to be transcribed at a high frequency, and a poly-A recognition sequence at the other end for proper processing and transport of the messenger RNA. An example of such a preferred (empty) expression cassette into which the DNA sequence of the present invention can be inserted is the pPHI414 plasmid developed by Beach et al. of Pioneer Hi-Bred International, Inc., Johnston, IA. Highly preferred plant expression cassettes will be designed to include one or more selectable marker genes, such as kanamycin resistance or herbicide tolerance genes.

By the term "vector" herein is meant a DNA sequence which is able to replicate and express a foreign gene in a host cell. Typically, the vector has one or more endo-nuclease recognition sites which may be cut in a predictable fashion by use of the appropriate enzyme. Such vectors are preferably constructed to include additional structural gene sequences imparting antibiotic or herbicide resistance, which then serve as selectable markers to identify and separate transformed cells. Preferred selection agents include kanamycin, chlorosulfuron, phosphonothricin, hygromycin and methotrexate, and preferred markers are genes conferring resistance to these compounds. A cell in which the foreign genetic material in a vector is functionally expressed has been "transformed" by the vector and is referred to as a "transformant".

A particularly preferred vector is a plasmid, by which is meant a circular double-stranded DNA molecule that is not a part of the chromosomes of the cell.

As mentioned above, genomic, synthetic and cDNA encoding the limonene synthase enzyme, and as necessary genomic, synthetic and cDNA encoding the GPP synthase enzyme, may be used in this invention. The vector of interest may also be constructed partially from a cDNA clone, partially from a synthetic sequence and partially from a genomic clone, or a combination thereof. When the gene sequence or sequences is/are in hand, genetic constructs are made which contain the necessary regulatory sequences to provide for efficient expression of the gene in the host cell. According to this invention, the genetic construct will contain (a) at least one genetic sequence coding for the enzyme responsible for making the larvicidal compound of interest and (b) one or more regulatory sequences operably linked on either side of the structural gene or genes. Typically, the regulatory sequences will be selected from the group comprising of promoters and terminators. The regulatory sequences may be from autologous or heterologous sources. In preferred embodiments involving the use of both the limonene synthase gene and the GPP synthase gene, each gene will be under the control of its own regulatory sequences.

Promoters that may be used in the genetic sequence include nos, ocs, phaseolin, CaMV, FMV, ubiquitin, and other promoters isolated from the DNA of plants or other sources, both natural and synthetic.

An efficient plant promoter that may be used is an overproducing plant promoter. Overproducing plant promoters that may be used in this invention include the promoter of the small sub-unit (ss) of the ribulose-1,5-bisphosphate carboxylase from soybean (Berry-Lowe et al, J. Molecular and App. Gen., 1:483-498 (1982)), and the promoter of the cholorophyll a-b binding protein. However, these two promoters are known to be light-induced in eukaryotic plant cells (see, for example, Genetic Engineering of Plants, An Agricultural Perspective, Cashmore, Pelham, New York, 1983, pp. 29-38, G. Coruzzi et al., J. Biol. Chem., 258:1399 (1983), and P. Dunsmuir, et al., J. Molecular and App. Gen., 2:285 (1983)) and may be less desirable when root expression is desired. An especially preferred constitutive promoter is the 35S promoter from Cauliflower Mosaic Virus.

Root-specific promoters are especially preferred for the control of Southern corn rootworm while minimizing limonene production in the agronomically valuable parts of the plant. Such promoters are also known and can be selected from the many available from the literature or isolated de novo from various compatible species. For example, Hirel, B., Marsolier, M.C., Hoarau, A., Hoarau, J., Brangeon, J., Schafer, R., and Verma, D.P.S., Plant Molecular Biology, Oct 1992. v. 20 (2) p. 207-218, describe a root-specific glutamine synthetase gene from soybean. Keller, B. and Baumgartner, C., The Plant Cell, Oct 1991. v. 3 (10) p. 1051-1061, describe a root-specific control element in the GRP 1.8 gene of French bean. Sanger, M., Daubert, S., and Goodman, R.M., Plant Molecular Biology Mar 1990. v. 14 (3) p. 433-443, discuss the root-specific promoter of the Mannopine Synthase (MAS) gene of *Agrobacterium tumefaciens*. Miao, G.H., Hirel, B., Marsolier, M.C., Ridge, R.W., and Verma, D.P.S., The Plant Cell, Jan 1991. v. 3 (1) p. 11-22, describe a full-length cDNA clone encoding cytosolic glutamine synthetase (GS), which is expressed in roots and root nodules of soybean. Bogusz, D., Llewellyn, D.J., Craig, S., Dennis, E.S., Appleby, C.A., and Peacock, W.J., The Plant Cell, July 1990. v. 2 (7) p. 633-641, discusses two root-specific promoters isolated from hemoglobin genes from the nitrogen-fixing nonlegume *Parasponia andersonii* and the related non-nitrogen-fixing nonlegume *Trema tomentosa*. The promoters of these genes were linked to a beta-glucuronidase reporter gene and introduced into both the nonlegume *Nicotiana tabacum* and the legume *Lotus corniculatus*, and in both instances root-specific promoter activity was preserved. Leach, F. and Aoyagi, K., Plant Science (Limerick) 1991, 79 (1): 69-76, describe their analysis of the promoters of the highly expressed rolC and rolD root-inducing genes of *Agrobacterium rhizogenes*. They concluded that enhancer and tissue-specific DNA determinants are dissociated in those promoters. Teeri, T. H., Lehvaslaiho, H., Franck, M., Uotila, J., Heino, P., Palva, E. T., Montagu, M. van, and Herrera-Estrella, L., EMBO Journal, 1989, 8 (2): 343-350, used gene fusions to lacZ to show that the *Agrobacterium* T-DNA gene encoding octopine synthase is especially active in the epidermis of the root tip and that the TR2' gene was root specific in the intact plant and stimulated by wounding in leaf tissue, an especially desirable combination of characteristics for use with an insecticidal or larvicidal gene. The TR1' gene, fused to NPTII, (neomycin phosphotransferase II) showed similar characteristics.

The expression cassette comprising the structural gene or genes of the method of the invention operably linked to the desired control sequences can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells. Typically, genes conferring resistance to antibiotics or selected herbicides are used. After the genetic material is introduced into the target cells, successfully transformed cells and/or colonies of cells can be isolated by selection on the basis of these markers.

Typically, an intermediate host cell will be used in the practice of this invention to increase the copy number of the cloning vector. With an increased copy number, the vector containing the gene of interest can be isolated in significant quantities for introduction into the desired plant cells. Host cells that can be used in the practice of this invention include prokaryotes, including bacterial hosts such as *E. coli, S. typhimurium*, and *S. marcescens*. Eukaryotic hosts such as yeast or filamentous fungi may also be used in this invention.

The isolated cloning vector will then be introduced into the plant cell using any convenient technique, including electroporation (in protoplasts), retroviruses, microparticle bombardment, and microinjection, into cells from monocotyledonous or dicotyledonous plants, in cell or tissue culture, to provide transformed plant cells containing as foreign DNA at least one copy of the DNA sequence or sequences of the plant expression cassette. Preferably, the monocotyledonous species will be selected from maize, sorghum, wheat and rice, and the dicotyledonous species will be selected from soybean, sunflower, cotton, rapeseed (either edible or industrial), alfalfa, tobacco, and Solanaceae such as potato and tomato. Using known techniques, protoplasts can be regenerated and cell or tissue culture can be regenerated to form whole fertile plants which carry and express the desired gene for the selected protein. Accordingly, a highly preferred embodiment of the present invention is a transformed maize plant, the cells of which contain as foreign DNA at least one copy of the DNA sequence or sequences of an expression cassette which drives expression of limonene synthase, and in some instances GPP synthase as well.

This invention also provides methods of imparting resistance to *Diabrotica undecimpunctata howardi* to plants of a susceptible taxon, comprising the steps of:

a) culturing cells or tissues from at least one plant from the taxon,

b) introducing into the cells of the cell or tissue culture at least one copy of an expression cassette comprising a structural gene coding for either limonene synthase or both limonene synthase and GPP synthase, operably linked to plant regulatory sequences which cause the expression of the enzyme in the cells, and

c) regenerating insect-resistant whole plants from the cell or tissue culture. Once whole plants have been obtained, they can be sexually or clonally reproduced in such manner that at least one copy of the sequence provided by the expression cassette is present in the cells of progeny of the reproduction.

Alternatively, once a single transformed plant has been obtained by the foregoing recombinant DNA method, conventional plant breeding methods can be used to transfer the structural gene or genes and associated regulatory sequences via crossing and backcrossing. Such intermediate methods will comprise the further steps of

a) sexually crossing the insect-resistant plant with a plant from the insect-susceptible taxon;

b) recovering reproductive material from the progeny of the cross; and

c) growing insect-resistant plants from the reproductive material. Where desirable or necessary, the agronomic characteristics of the susceptible taxon can be substantially preserved by expanding this method to include the further steps of repetitively:

a) backcrossing the insect-resistant progeny with insect-susceptible plants from the susceptible taxon; and

b) selecting for expression of insect resistance (or an associated marker gene) or limonene production among the progeny of the backcross, until the desired percentage of the characteristics of the susceptible taxon are present in the progeny along with the gene imparting insect resistance.

By the term "taxon" herein is meant a unit of botanical classification of genus or lower. It thus includes genus, species, cultivars, varieties, variants, and other minor taxonomic groups which lack a consistent nomenclature.

It will also be appreciated by those of ordinary skill that the plant vectors provided herein can be incorporated into *Agrobacterium tumefaciens*, which can then be used to transfer the vector into susceptible plant cells, primarily from dicotyledonous species. Thus, this invention provides a method for imparting insect resistance in *Agrobacterium tumefaciens*-susceptible dicotyledonous plants in which the expression cassette is introduced into the cells by infecting the cells with *Agrobacterium tumefaciens*, a plasmid of which has been modified to include a plant expression cassette which expresses limonene synthase in the manner of this invention.

Finally, insect pests of harvested material, including stored grain, can also be targets for the methods of this invention. In view of this, the invention also provides methods for killing larvae of *Diabrotica undecimpunctata howardi* in harvested materials and products obtained from harvested materials, comprising applying limonene to the grain or causing either limonene synthase or both GPP synthase and limonene synthase to be expressed in the grain.

The following description further exemplifies the compositions of this invention and the methods of making and using them. However, it will be understood that other methods, known by those of ordinary skill in the art to be equivalent, can also be employed.

## EXAMPLES 1-6

### Insect larvae inhibition and toxicity assays For Southern Corn Rootworm (SCR) and European Corn Borer (ECB)

Bioassay diets were prepared as described in Czapla and Lang in "Effect of Plant Lectins on the Larval Development of European Corn Borer (Lepidoptera: Pyralidae) and Southern Corn Rootworm (Coleoptera: Chrysomelidae)", J. Econ. Entomol., 83:2480-85 (1990), except that low melting temperature agarose replaced the regular agarose so that the diets could be chilled to 37°C prior to the addition of limonene (one assay used the regular agarose diet).

Results were as follows. In Examples 1-4, the test larvae were Southern Corn Rootworm. In Examples 5-6, the test larvae were European Corn Borer. The results of each experiment represent the average from 16-32 insects. All limonene concentrations (ppm) are by weight. The SCR data indicate that limonene is effective against the larvae, but when limonene was used in the same protocol against ECB, little or no effect was seen, indicating that limonene is ineffective against some common, relatively susceptible crop pests.

$$\% \text{ Corr. Mortality} = 100 \text{ x} \left| \frac{\text{mortality of treated } - \text{ control}}{100 - \text{control}} \right|$$

$$\% \text{ Wt. Reduction} = 100 \text{ x} \left| \frac{\text{control wt.} - \text{treated wt.}}{\text{control wt}} \right|$$

| Example: | | 1 | | 2 | |
|---|---|---|---|---|---|
| Limonene ppm | % Corr. Mortal. | % weight Reduction | % Corr. Mortal. | % weight Reduction |
| 10,000 | 57 | 64 | 80 | 86 |
| 1,000 | 49 | 52 | 19 | 30 |
| 100 | 57 | 52 | 15 | 6 |

| Example: | | 3 | | 4 | |
|---|---|---|---|---|---|
| Limonene ppm | % Corr. Mortal. | % weight Reduction | % Corr. Mortal. | % weight Reduction |
| | 96 | 64 | 44 | 0 |
| | 7 | 0 | 22 | 0 |
| | 26 | 0 | 19 | 0 |

Average SCR Results:

| Limonene ppm | % Corr. Mortal. | % weight Reduction |
|---|---|---|
| 10,000 | 69 | 54 |
| 1,000 | 24 | 21 |
| 100 | 29 | 15 |

| Example: | | 5 | | 6 | |
|---|---|---|---|---|---|
| Limonene ppm | % Corr. Mortal. | % weight Reduction | % Corr. Mortal. | % weight Reduction |
| 10,000 | 0 | 28 | 0 | 0 |
| 1,000 | 0 | 16 | 0 | 0 |
| 100 | 0 | 1 | 0 | 7 |

Average ECB Results:

| Limonene ppm | % Corr. Mortal. | % weight Reduction |
|---|---|---|
| 10,000 | 0 | 14 |
| 1,000 | 0 | 8 |
| 100 | 0 | 4 |

## EXAMPLE 7

Maize callus cultures were transformed by microprojectile bombardment using plasmids containing a cloned gene coding for the limonene synthase (limonene cylase) enzyme driven by a ubiquitin promoter and a ubiquitin intron and followed downstream by a PIN-II terminator. Whole, fertile plants were regenerated from the transformed callus and analyzed for limonene synthase and limonene. Representative results from one series were as follows:

| Clone # | Callus LS[1] | Callus Limonene[2] | Plant | Plant LS | Plant Limonene |
|---------|---------|----------------|-------|----------|----------------|
| C6 | 22539 | ND | 3 | 1950 | ND |
| | | | 7 | 2000 | ND |
| C19 | 9900 | ND | 6 | 2100 | ND |
| C3 | 2550 | ND | 3 | 1900 | ND |
| | | | 4 | 1650 | ND |

[1]LS = Limonene Synthase
[2]ND = not detected above 0.5 ng/g fresh wt.

In other experiments, transgenic maize plants were produced and tissues that exhibited high expression of the limonene synthase protein. The enzyme was extracted from transformed plants and tissues and allowed to react with tritium-labeled geranyl pyrophosphate (GPP). The extracted enzyme converted GPP to limonene, showing that a functional enzyme was being produced. In addition, western blots were done and confirmed the presence of LS protein in transgenic tissues but not in negative control tissues. Seed was collected from the transformed plants. In sum, whole fertile, transformed plants have been produced that express active limonene cyclase (synthase) according to the teaching of the above-identified application.

## EXAMPLE 8

In view of the results set forth, in Example 7, demonstrating expression of active limonene synthase in whole, fertile, transformed plants, larvicidally effective amounts of limonene can be produced in such transgenic plants where sufficient substrate is present for the limonene synthase enzyme to act on. Therefore, maize callus cultures can be transformed by art recognized microprojectile bombardment methods using plasmids containing genes coding for both the limonene synthase enzyme and the GPP synthase enzyme, driven, for example, by one or more promoters (a ubiquitin promoter, for example) and followed downstream by, for example, a PIN-II terminator. Whole, fertile plants are regenerated from the transformed callus and analyzed for presence of and/or activity of both enzymes, and are also analyzed for the presence of limonene.

Alternatively, callus can be generated from transgenic plants that contain and express the limonene synthase transgene, and such callus cultures can be transformed as described above, except that such callus can be transformed using plasmids containing a gene coding for the GPP synthase protein. The whole, fertile, transgenic plants regenerated from such transformed callus would be expected to produce larvicidally effective amounts of limonene.

## Claims

1.   A method for protecting a monocotyledonous plant against infestation by larvae of *Diabrotica* spp comprising inserting into the genome of the plant at least one nucleotide sequence coding for limonene cyclase and at least one nucleotide sequence coding for GPP synthase, each of said sequences in proper reading frame relative to transcription initiator and promoter sequences active in the plant to cause expression of the enzymes at levels that provide for production of a plant protective effective amount of limonene in the tissues of the plant.

2.   The method of claim 1 wherein the monocotyledonous plant is a corn plant (*Zea mays* L).

3.   The method of claim 1 or 2 wherein one or both of the promoters are tissue-specific promoters.

4.   The method of claim 3 wherein one or both of the promoters are root-specific promoters.

5.   The method of any preceding claim, further comprising:

(a) crossing the insect-resistant plant thus obtained with a plant from an insect-susceptible taxon;

(b) recovering reproductive material from the progeny of the cross; and

(c) growing further insect-resistant plants from the reproductive material;
and optionally

(d) repetitively backcrossing the insect-resistant progeny with insect-susceptible plants from the susceptible taxon and selecting for expression of insect resistance, or an associated marker gene, or limonene production among the progeny of the backcross, until the desired percentage of the characteristics of the susceptible taxon are present in the progeny along with the limonene cyclase and GPP synthase sequences.

**Patentansprüche**

1. Verfahren zum Schutz einer monocotylen Pflanze gegen Befall durch Larven von *Diabrotica spp*, umfassend die Insertion mindestens einer Nukleotidsequenz, die Limonencyclase codiert, und mindestens einer Nukleotidsequenz, die GPP-Synthase codiert, in das Genom der Pflanze, wobei jede der Sequenzen in geeigneten Leseraster, bezogen auf die Transkriptionsinitiator- und Promotorsequenzen, die in der Pflanze aktiv sind, vorliegt, um die Expression der Enzyme in Gehalten zu verursachen, die die Produktion einer die Pflanze schützenden wirksamen Menge an Limonen in dem Gewebe der Pflanze ergeben.

2. Verfahren nach Anspruch 1, wobei die monocotyle Pflanze eine Maispflanze (*Zea mays L.*) ist.

3. Verfahren nach Anspruch 1 oder 2, wobei einer oder beide der Promotoren gewebsspezifische Promotoren sind.

4. Verfahren nach Anspruch 3, wobei einer oder beide der Promotoren wurzelspezifische Promotoren sind.

5. Verfahren nach einem der vorstehenden Ansprüche, umfassend weiter:

(a) Kreuzung der so erhaltenen insektenresistenten Pflanze mit einer Pflanze aus einem insektenempfänglichen Taxon;

(b) Isolieren von reproduktivem Material aus den Nachkommen der Kreuzung; und

(c) Züchten weiterer insektenresistenter Pflanzen aus dem reproduktiven Material;
und gegebenenfalls

(d) wiederholtes Rückkreuzen der insektenresistenten Nachkommen mit insektenempfänglichen Pflanzen aus dem empfänglichen Taxon und Selektion der Expression von Insektenresistenz oder eines assoziierten Markergens oder Limonenproduktion unter den Nachkommen der Rückkreuzung, bis der gewünschte Prozentsatz der Eigenschaften des empfänglichen Taxons in den Nachkommen zusammen mit den Limononencyclase- und GPP-Synthasesequenzen vorhanden ist.

**Revendications**

1. Méthode destinée à protéger une plante Monocotylédone contre l'infestation par des larves de *Diabrotica* ssp comprenant l'insertion dans le génome de la plante d'au moins une séquence nucléotidique codant pour la limonène cyclase et au moins une séquence nucléotidique codant pour la GPP synthase, chacune desdites séquences dans le cadre de lecture correct par rapport à l'initiation de la transcription et des séquences promotrices actives dans la plante pour entraîner l'expression des enzymes à des niveaux permettant la production d'une quantité de limonène dans les tissus de la plante, assurant une protection efficace de la plante.

2. Méthode selon la revendication 1 dans laquelle la plante Monocotylédone est le maïs (*Zea mays* L.).

3. Méthode selon la revendication 1 ou 2 dans laquelle un des deux promoteurs ou les deux sont des promoteurs tissu-spécifiques.

4. Méthode selon la revendication 3 dans laquelle un des deux promoteurs ou les deux sont spécifiques de la racine.

5. Méthode selon l'une quelconque des revendications précédentes, comprenant en outre :

(a) le croisement de la plante résistante aux insectes ainsi obtenue avec une plante d'un taxon sensible aux insectes ;

(b) la récolte de matériel reproducteur à partir de la descendance du croisement ; et

(c) la culture par la suite de plantes résistantes aux insectes à partir du matériel reproducteur ;

et éventuellement

(d) la réalisation de rétrocroisements (backcross) répétitifs de la descendance résistante aux insectes avec des plantes sensibles aux insectes à partir du taxon sensible et la sélection de l'expression de la résistance aux insectes, ou d'un gène marqueur associé, ou de la production de limonène dans la descendance du rétrocroisement, jusqu'à ce que le pourcentage désiré de caractéristiques du taxon sensible soit présent dans la descendance en même temps que les séquences de la limonène cyclase et de la GPP synthase.